# EUROPÄISCHE PATENTSCHRIFT

(11) **EP 0 708 083 B1**
(45) Veröffentlichungstag und Bekanntmachung des Hinweises auf die Patenterteilung: **19.08.1998**
(21) Anmeldenummer: 95116248.6
(22) Anmeldetag: 16.10.1995
(51) Int. Cl.: C07C 249/04, C07C 251/60

(54) **Verfahren zur Herstellung von Isopropylidenamino-oxyessigsäure(methoxycarbonylmethylen)ester**
Process for preparing isopropylidene amino-oxy-acetic acid (methoxycarbonylmethylene) ester
Procédé pour la préparation de l'ester méthoxycarbonylméthylémique de l'acide isopropylidèneaminooxyacétique

(30) Priorität: 22.10.1994 DE 4437904
(43) Veröffentlichungstag der Anmeldung: 24.04.1996
(73) Patentinhaber: BASF AKTIENGESELLSCHAFT, 67056 Ludwigshafen (DE)
(72) Erfinder: Keil, Michael, Dr., D-67251 Freinsheim (DE); Wahl, Josef, D-67105 Schifferstadt (DE)

(56) Entgegenhaltungen:
- EP-A- 0 023 560
- EP-A- 0 243 834

## Beschreibung

Die vorliegende Erfindung betrifft ein Verfahren zur Herstellung von Isopropylidenaminooxyessigsäure(methoxycarbonylmethylen)ester der Formel I

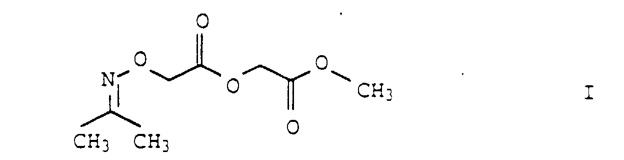

Verbindung I dient als Bioregulator zur Senkung des endogenen Ethylenspiegels in Pflanzen (EP-A 243 834).

Gemäß dem Stand der Technik kann Verbindung I über die im nachfolgenden Schema dargestellten Einzelschritte 1) bis 3) erhalten werden.

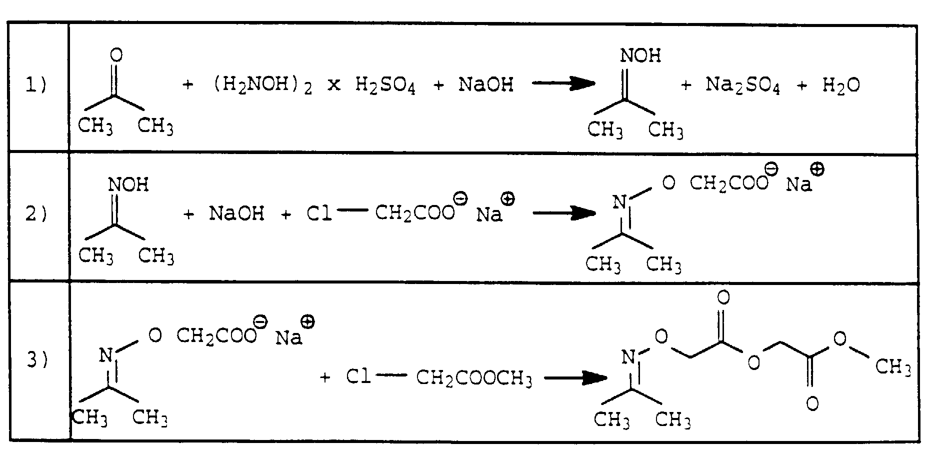

Die Herstellung von Acetonoxim ist z.B. beschrieben in Houben-Weyl, Methoden der Organischen Chemie, Bd. 10/4, S. 58, 1968. Die Alkylierung mit Chloressigsäure-Na-Salz ist in Zhurn. Obshch. Khim. 52 (1), 223, 1982, bzw. in WO 89/11473 und EP-A 158 159 offenbart. Die anschließende Alkylierung mit Chloressigsäuremethylester ist EP-A 243 834 zu entnehmen.

Die Umsetzung der beschriebenen 3 Stufen zu einem wirtschaftlichen Prozeß in der Produktion von großen Mengen beinhaltet jedoch folgende Schwierigkeiten:
1. Die Isolierung und Reinigung der Zwischenstufen durch Filtration oder Destillation ist aufwendig und teuer. Die Gesamtausbeute über alle Stufen ist unbefriedigend.
2. Die Alkylierung von Acetonoxim in protischen Lösungsmitteln (Zhurn. Obshch. Khim 52 (1), 223 bzw. EP-A 121 701) verläuft nur in unbefriedigenden Ausbeuten, die beschriebenen Umsetzungen in aprotischen Lösungsmitteln (EP-A 158 159 bzw. WO 89/11473) erforderten einen großen Überschuß an Oxim.

Es besteht daher Bedarf an einem einfachen Verfahren, bei dem
- auf Reinigungsschritte von Zwischenstufen verzichtet werden kann,
- auf einen Überschuß an Acetonoxim verzichtet werden kann,
- alle Stufen prinzipiell in einem Reaktionskessel durchgeführt werden können.

Verbunden mit der Realisierung dieser Anforderungen wäre ein geringerer Apparateaufwand, eine günstigere Gesamtausbeute und damit eine höhere Wirtschaftlichkeit.

Demgemäß wurde nun ein Verfahren zur Herstellung von Isopropylidenaminooxyessigsäurelmethoxycarbonylmethylen)ester der Formel I 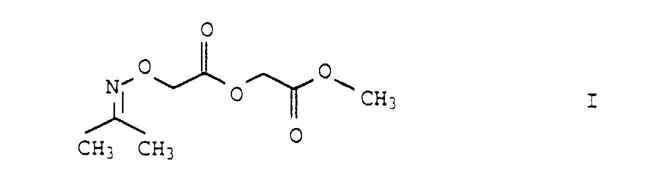 gefunden, das dadurch gekennzeichnet ist, daß man in einem integrierten Prozeß, ohne Isolierung von Zwischenstufen
a) Aceton mit Hydroxylammoniumsulfat und Natronlauge zum Acetonoxim umsetzt, wobei die Umsetzung in Gegenwart von Toluol vorgenommen und/oder das Acetonoxim aus dem Reaktionsgemisch mit Toluol extrahiert wird,
b) die so erhaltene toluolische Lösung mit 1,0 bis 1.5 mol, bezogen auf Acetonoxim, Natronlauge versetzt und Wasser auskreist,
c) Toluol destillativ gegen ein dipolar aprotisches Lösungsmittel austauscht,
d) die so erhaltene Kristallmaische mit dem Natriumsalz von Chloressigsäure zum Natriumsalz der Isopropylidenaminooxyessigsäure umsetzt und das Reaktionsgemisch anschließend mit Chloressigsäuremethylester versetzt und
e) den Isopropylidenaminooxyessigsaure(methoxycarbonylmethylen)ester in an sich bekannter Weise isoliert,

Der erste Reaktionsschritt (Schritt a)) im erfindungsgemäßen Eintopfverfahren wird in an sich bekannter Weise in wäßrigem Medium durchgeführt und dann das Acetonoxim mit Toluol aus dem Reaktionsgemisch extrahiert oder vorteilhaft die Umsetzung bereits in Gegenwart von Toluol vorgenommen. Dabei liegt die Menge an Toluol zweckmäßigerweise bei etwa 150 bis 900 ml, bezogen auf 1 mol gebildetes Acetonoxim.

Die so erhaltene Reaktionsmischung wird ohne weitere Reinigung mit Natronlauge versetzt. Bei der so gestalteten Reaktionsführung sind für die anschließende Methylierung überraschenderweise keine hohen Überschüsse an Acetonoxim erforderlich. Stattdessen werden 1,0 bis 1,5 mol, insbesondere 1,0 bis 1,2 mol, Natronlauge bezogen auf Acetonoxim, zugegeben.

Bei der Salzbildung entstehendes Reaktionswasser und durch Natronlauge eingeschlepptes Wasser werden während der Umsetzung oder danach azeotrop aus dem Reaktionsgemisch entfernt, beispielsweise bei Temperaturen von 50 bis 110°C und Drücken zwischen 100 mbar und 1 bar.

Anschließend wird ein Lösungsmittelwechsel vorgenommen, z.B. indem man die toluolische Suspension mit einem geeigneten dipolar aprotischen Lösungsmittel wie z.B. N-Methylpyrrolidon oder insbesondere Dimethylformamid, die das Acetonoxim-Na-salz besser anlösen und zur Reaktion bringen können, versetzt. Gleichzeitig oder anschließend wird Toluol destillativ entfernt.

Die Menge an zugesetztem dipolar aprotischen Lösungsmittel entspricht zweckmäßigerweise den Mengen an abdestilliertem Toluol, d.h. liegt etwa zwischen 150 bis 900 ml pro mol Acetonoxim-Na-salz.

Die so erhaltene Kristallmaische wird im nächsten Schritt (Schritt d)) zunächst mit dem Natriumsalz von Chloressigsäure(etwa 1 Moläquivalent umgesetzt und dann aus dem Natriumsalz der Isopropylidenaminoessigsäure mit Chloressigsäuremethylester das Produkt I gebildet. Der erste Reaktionsschritt wird beispielsweise bei Temperaturen von 20 bis 150°C, insbesondere 50 bis 100°C vorgenommen. Für den letzten Reaktionsschritt haben sich Temperaturen von 20 bis 150°C, insbesondere 50 bis 80°C, als zweckmäßig erwiesen. Die Reaktionspartner können in etwa stöchiometrischen Menge, d.h. Mengen von 1 bis 1,2 mol pro Mol Na-salz vorliegen. Ein höherer Überschuß des einen oder anderen Reaktionspartners wäre möglich, jedoch kaum wirtschaftlich.

Das Wertprodukt I kann in an sich bekannter Weise aus dem Reaktionsgemisch abgetrennt werden. Als besonders vorteilhaft hat sich die folgende Aufarbeitung erwiesen: Man destilliert das Lösungsmittel bei 50 bis 80°C und 20 bis 200 mbar ab, nimmt den Rückstand in Toluol auf, wäscht das Gemisch mit Wasser und destilliert fraktioniert.

Die folgende Beschreibung verdeutlicht das beanspruchte Verfahren. Die erzielte Gesamtausbeute ist überraschend hoch und die Versuchsdurchführung wesentlich einfacher als die Maßnahmen bei schrittweiser Herstellung der Zwischenstufen.

### Beispiel

### Herstellung von Isopropylidenaminooxyessigsäure(methoxycarbonylmethylen)ester

980 ml Wasser, 328 g Hydroxylammoniumsulfat und 460 ml Toluol, werden bei 20 bis 50°C vorgelegt und bei pH 4,5 bis 5,0 232 g (4 mol) Aceton sowie 320 g (4 mol) konz. Natronlauge gleichzeitig zugetropft. Zur Vervollständigung der Reaktion wird ca. 1/2 Std. nachgerührt und bei 30 bis 40°C die Phasen getrennt. Die Wasserphase wird 2x mit 460 ml Toluol ausgeschüttelt. Die gesamte organische Phase wird unter 100 - 200 mbar Vakuum zum Rückfluß erhitzt (55°C). Zur Salzbildung tropft man 320 g (4 mol) konz. Natronlauge zu und kreist 250 g Wasser gleichzeitig aus. Anschließend wird bei gleicher Temperatur Toluol abdestilliert und 2000 ml DMF zugefahren. In die Kristallmaische gibt man bei 50°C in Portionen 466 g (4 mol) Chloressigsäure-Na-Salz zu und rührt 5 Std. bei 50°C. Anschließend tropft man bei 50 bis 55°C 433 g (4 mol) Chloressigsäuremethylester zu, rührt 10 Std. bei 55°C weiter und destilliert dann bei gleicher Temperatur und Vakuum DMF über. Der Rückstand wird in Toluol aufgenommen und mit Wasser gewaschen. Nach einer fraktionierten Destillation bei 100°C/0,2 Torr erhält man 531 g Endprodukt. Dies entspricht einer Gesamtausbeute über alle Stufen von 65,3 % d.Th. Das Produkt wird in einer GC-Reinheit von 99,9 % erhalten und weist einen Brechungsindex n_{D}²⁰ von 1,4469 auf.

### Vergleichende Betrachtung zu Literaturausbeuten:

Die Herstellung von Acetonoxim gelingt mit ca. 90 % Ausbeute. Die Alkylierung mit Chloressigsäure-Na-Salz ist beschrieben mit 49 % Ausbeute (Zhurn. Obshch. Khim. 52 (1),223), Bei den analogen Verfahren in WO 89/11473 bzw. EP-A 158 159 werden zwar Ausbeuten um 80 % erzielt, allerdings nur unter Verwendung eines großen Überschusses an Oxim. Der zweite Alkylierungsschritt erfolgt gemäß EP-A 234 834 mit Bromessigester in 75 % Ausbeute. Bei Verwendung von Chloressigsäuremethylester werden noch geringere Ausbeuten erhalten.

## Patentansprüche

1. Verfahren zur Herstellung von Isopropylidenaminooxyessigsäure(methoxycarbonylmethylen)ester der Formel I 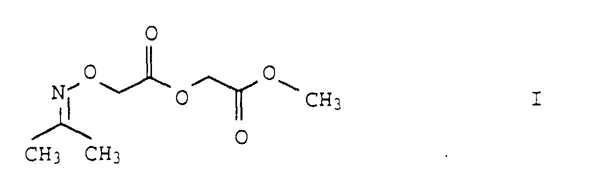 dadurch gekennzeichnet, daß man in einem integrierten Prozeß, ohne Isolierung von Zwischenstufen
a) Aceton mit Hydroxylammoniumsulfat und Natronlauge zum Acetonoxim umsetzt, wobei die Umsetzung in Gegenwart von Toluol vorgenommen und/oder das Acetonoxim aus dem Reaktionsgemisch mit Toluol extrahiert wird,
b) die so erhaltene toluolische Lösung mit 1,0 bis 1,5 mol, bezogen auf Acetonoxim, Natronlauge versetzt und Wasser auskreist,
c) Toluol destillativ gegen ein dipolar aprotisches Lösungsmittel austauscht,
d) die so erhaltene Kristallmaische mit dem Natriumsalz von Chloressigsäure zum Natriumsalz der Isopropylidenaminooxyessigsäure umsetzt und das Reaktionsgemisch anschließend mit Chloressigsäuremethylester versetzt und
e) den Isopropylidenaminooxyessigsäure (methoxycarbonylmethylen)ester in an sich bekannter Weise isoliert.

2. Verfahren nach Anspruch 1, dadurch gekennzeichnet, daß man als dipolar aprotisches Lösungsmittel in Stufe c) Dimethylformamid verwendet.

3. Verfahren nach Anspruch 1, dadurch gekennzeichnet, daß man die Verbindung I aus dem Reaktionsgemisch isoliert, indem man das Lösungsmittel abdestilliert, den Rückstand mit Toluol versetzt, mit Wasser wäscht und destilliert.

## Claims

1. A process for preparing methyl isopropylideneaminooxyacetoxyacetate of the formula I 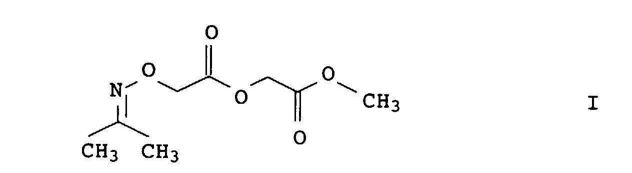 which comprises reacting in an integrated process, without isolation of intermediates,
a) acetone with hydroxylammonium sulfate and sodium hydroxide solution to give acetone oxime, the reaction being performed in the presence of toluene and/or the acetone oxime being extracted from the reaction mixture with toluene,
b) treating the toluene solution thus obtained with from 1.0 to 1.5 mol, based on acetone oxime, of sodium hydroxide solution and removing water,
c) exchanging toluene for a dipolar aprotic solvent by distillation,
d) reacting the crystal mash thus obtained with the sodium salt of chloroacetic acid to give the sodium salt of isopropylideneaminooxyacetic acid and then treating the reaction mixture with methyl chloroacetate and
e) isolating the methyl isopropylideneaminooxyacetoxyacetate in a manner known per se.

2. A process as claimed in claim 1, wherein the dipolar aprotic solvent used in step c) is dimethylformamide.

3. A process as claimed in claim 1, wherein the compound I is isolated from the reaction mixture by distilling off the solvent, treating the residue with toluene, washing with water and distilling.

## Revendications

1. Procédé pour la préparation d'ester méthoxycarbonylméthylénique d'acide isopropylidèneaminooxyacétique de formule I 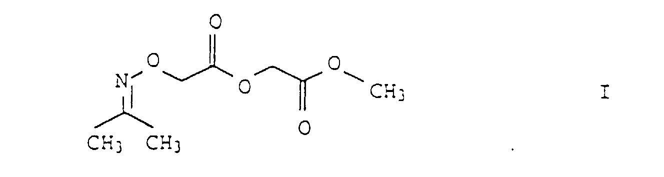 caractérisé par le fait que, dans un procédé intégré, sans isolement d'étapes intermédiaires,
a) on fait réagir de l'acétone avec du sulfate d'hydroxylammonium et de la lessive de soude pour former un acétonoxime, la réaction étant effectuée en présence de toluène et/ou l'acétonoxime étant extrait du mélange réactionnel avec du toluène,
b) on ajoute à la solution toluénique ainsi obtenue 1,0 à 1,5 mol, par rapport à l'acétonoxime, de lessive de soude et on élimine l'eau,
c) on remplace le toluène par distillation par un solvant aprotique dipolaire,
d) on fait réagir la masse cristalline ainsi obtenue avec le sel de sodium de l'acide chloroacétique pour former le sel de sodium de l'acide isopropylidèneaminooxyacétique et on ajoute ensuite au mélange réactionnel de l'ester méthylique d'acide chloroacétique et
e) on isole l'ester méthoxycarbonylméthylénique de l'acide isopropylidèneaminooxyacétique d'une manière connue en soi.

2. Procédé selon la revendication 1, caractérisé par le fait qu'on utilise du diméthylformamide comme solvant aprotique dipolaire dans l'étape c).

3. Procédé selon la revendication 1, caractérisé par le fait qu'on isole le composé I du mélange réactionnel, tandis qu'on élimine le solvant par distillation, on ajoute du toluène au résidu, on lave avec de l'eau et on distille.
